# EUROPÄISCHE PATENTSCHRIFT

(11) **EP 2 969 029 B1**
(45) Veröffentlichungstag und Bekanntmachung des Hinweises auf die Patenterteilung: **11.07.2018**
(21) Anmeldenummer: 14715850.5
(22) Anmeldetag: 14.02.2014
(51) Int. Cl.: A61Q 19/02, A61K 8/36, A61K 8/365, A61K 8/49

(54) **VERWENDUNG KOSMETISCH ODER DERMATOLOGISCH UNBEDENKLICHER SUBSTITUIERTER MICHAEL-AKZEPTOREN ZUR VERHINDERUNG, VERMINDERUNG ODER PROPHYLAXE DER TYROSINASEAKTIVITÄT DER MENSCHLICHEN HAUT UND/ODER DEREN AUFHELLUNG**
USE OF COSMETICALLY OR DERMATOLOGICALLY SAFE SUBSTITUTED MICHAEL ACCEPTORS FOR THE PREVENTION, REDUCTION OR PROPHYLAXIS OF THE TYROSINASE ACTIVITY OF THE HUMAN SKIN AND/OR THE LIGHTENING OF THE HUMAN SKIN
UTILISATION D'ACCEPTEURS DE MICHAËL SUBSTITUÉS COSMÉTIQUEMENT OU DERMATOLOGIQUEMENT ACCEPTABLES POUR LA SUPPRESSION, LA RÉDUCTION OU LA PROPHYLAXIE DE L'ACTIVITÉ DE TYROSINASE DE LA PEAU HUMAINE ET/OU DE SON ÉCLAIRCISSEMENT

(30) Priorität: 11.03.2013 DE 102013204070
(43) Veröffentlichungstag der Anmeldung: 20.01.2016
(73) Patentinhaber: Beiersdorf AG, 20253 Hamburg (DE)
(72) Erfinder: KOLBE, Ludger, 21255 Dohren (DE); SCHERNER, Cathrin, 22844 Norderstedt (DE); DINGLER, Christian, 22527 Hamburg (DE); BIERGIESSER, Helga, 21465 Reinbek (DE); MANN, Tobias, 22175 Hamburg (DE); GERWAT, Wolfram, 22393 Hamburg (DE)
(86) Internationale Anmeldenummer: PCT/EP2014/052962
(87) Internationale Veröffentlichungsnummer: WO 2014/139756

(56) Entgegenhaltungen:
- WO-A1-00/28961
- WO-A1-2004/073675
- WO-A1-2012/098134
- WO-A2-2006/078399
- WO-A2-2008/113495
- DE-A1-102010 045 890
- GB-A- 2 265 086
- JP-A- 2010 280 735
- US-A1- 2008 317 692
- ALISON J. WINDER ET AL: "New assays for the tyrosine hydroxylase and dopa oxidase activities of tyrosinase", EUROPEAN JOURNAL OF BIOCHEMISTRY, Bd. 198, Nr. 2, 1. Juni 1991 (1991-06-01), Seiten 317-326, XP055138592, ISSN: 0014-2956, DOI: 10.1111/j.1432-1033.1991.tb16018.x
- ROH JUNG SEOP ET AL: "Inhibitory effects of active compounds isolated from safflower (Carthamus tinctorius L.) seeds for melanogenesis", BIOLOGICAL & PHARMACEUTICAL BULLETIN (OF JAPAN), PHARMACEUTICAL SOCIETY OF JAPAN, TOKYO, JP, Bd. 27, Nr. 12, 1. Dezember 2004 (2004-12-01), Seiten 1976-1978, XP002577669, ISSN: 0918-6158
- WOO DUCK SEO ET AL: "Anti-pigmentation effect of serotonin alkaloid isolated from Korean barnyard millet (Echinochola utilis)", HAN'GUG EUNG'YONG SAENGMYEONG HWA HAGHOEJI - JOURNAL OF THE KOREAN SOCIETY FOR APPLIED BIOLOGICAL CHEMISTRY, vol. 55, no. 5, 29 September 2012 (2012-09-29), pages 579-586, XP55353545, Seoul, Korea ISSN: 1738-2203, DOI: 10.1007/s13765-012-2112-7

## Beschreibung

Die vorliegende Erfindung betrifft Verwendung kosmetisch oder dermatologisch unbedenklicher substituierter Michael-Akzeptoren zur Verhinderung, Verminderung oder Prophylaxe der Tyrosinaseaktivität der menschlichen Haut und/oder der Aufhellung der letzteren.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. - vernarbung (postinflammatorische Hyperpigmentierung) oder der Hautalterung (z.B. *Lentigines seniles*)*.*

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté - Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte "Triformula" entwickelt, die eine Kombination aus 0.1% Tretinoin, 5.0% Hydrochinon, 0.1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Weiterhin sind diverse andere Substanzen bekannt, für die eine hautaufhellende Wirksamkeit beschrieben wird. U.a. zu nennen sind hier Hexadecen-1,16-dicarbonsäure, Kojic-Säure und Derivate, Arbutin, Ascorbinsäure und Derivate, Flavonoide, Ellagsäure und Derivate, Tranexamsäure und verschiedene Resorcinol-Derivate, wie z.B. 4-n-Butylresorcin, 4-n-Hexylresorcin und 4-(1-phenylethyl)benzen-1,3-diol.

J.M. Ready beschreibt in einer Publikation (Bioorganic & Medicinal Chemistry Letter 17 (2007) 6871-6875 die Wirkung von u.a. substituierten Thiazol-Derivaten zur Inhibition der Mushroom tyrosinase.

In der Patentanmeldung der Firma Shiseido (WO 2009099195) werden substituierte Thiazolamine bzw. Hydrothiazolamine zur Hautaufhellung beschrieben.

Die im oben genannten Stand der Technik beschriebenen Substanzen weisen sich durch eine moderate Wirksamkeit und/oder eine schlechte galenische Stabilität aus.

Augenringe können ebenfalls als Folgen einer Pigmentierungsstörung entstehen, wobei sie ferner auch als Reaktion auf allgemeinen Stress, wie z.B. wenig Schlaf oder schlicht durch Überanstrengung der Augen erscheinen. Bei jüngeren Menschen verschwinden die Symptome nach ausreichender Nachtruhe wieder, über längere Zeiträume jedoch kann der Zustand chronisch und für die betroffenen Personen sehr störend werden. Auch gegen solche Hauterscheinungen mangelt es an genügend erfolgversprechenden Wirkstoffen und Behandlungsmöglichkeiten.

Ziel der nachfolgenden Erfindung war es also, dem nachteiligen Stand der Technik Abhilfe zu schaffen.
Gelöst wird diese Aufgabe durch die Verwendung kosmetisch oder dermatologisch unbedenklicher substituierter Michael-Akzeptoren zur Verhinderung, Verminderung oder Prophylaxe der Tyrosinaseaktivität der menschlichen Haut und/oder der Aufhellung der letzteren, wobei der Michael-Akzeptor oder die Michael-Akzeptoren gewählt wird oder werden aus der Gruppe

| |
|---|
| Benzyl cinnamat |
| Isoamyl p-Methoxycinnamat |
| Benzyl 2-hydroxybenzoat |
| Cyclohexyl 2-hydroxybenzoat |
| (E)-3-(thiophen-2-yl)acrylsäure |
| (E)-3-(pyridin-4-yl)acrylsäure |
| (E)-3-(pyridin-3-yl)acrylsäure |
| (E)-3-(1H-indol-3-yl)acrylsäure |
| (E)-3-(furan-2-yl)acrylsäure |
| 5-Propyl-2-thioxo-2,3-dihydropyrimidin-4(1H)-on |

Vorteilhaft enthalten kosmetische oder dermatologische Zubereitungen gemäß der erfindungsgemäßen Verwendung einen oder mehrere substituierte Michael-Akzeptoren, wobei die Gesamtmenge der substituierten Michael-Akzeptoren z. B. 0,000001 Gew.-% bis 30 Gew.-%, vorzugsweise 0,001 bis 15 Gew.-%, insbesondere 0,01 bis 5,0 Gew.-% beträgt, bezogen auf das Gesamtgewicht der Zubereitungen.

Vorteilhaft im Sinne der vorliegenden Erfindung folgt das Substtutionsmuster der erfindungsgemäß verwendeten Michael-Akzeptoren dem Schema der allgemeinen Formel 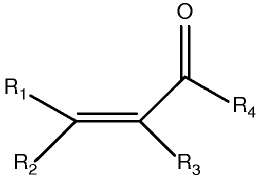 bei welcher
**R₁** = H, -OH, -Me und -Cl substituiert an Z, -Me, -OH und -OEt bedeuten kann,
**R₂** = -Phenyl (einfach oder mehrfach substituiert und unsubstituiert), - Alkyl, -Alkenyl, - Cycloalkyl, -zyklischer, aromatischer oder heteroaromatischer Rest (einfach oder mehrfach substituiert und unsubstituiert) verbunden mit R₃, - heteroaromatischer Rest, -zylischer Rest verbunden mit R₄ bedeuten kann,
**R₁ und R₂ =** ein zyklischer Rest Z : 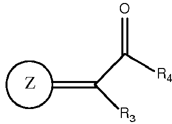 bedeuten kann,
**R₃** = -H, -Alkyl (linear oder verzweigt), -zyklischer, aromatischer oder heteroaromatischer Rest (einfach oder mehrfach substituiert und unsubstituiert) Rest verbunden mit R₂, -OH und -O-Glycosyl bedeuten kann,
**R₄** = -H, -Alkyl (einfach oder mehrfach substituiert und unsubstituiert), - Cycloalkyl (einfach oder mehrfach substituiert und unsubstituiert), -Aryl (einfach oder mehrfach substituiert und unsubstituiert), -OH, -O⁻, -OR₅, -zylischer Rest verbunden mit R₂, -NHR₅, -N(R₅)₂, bedeuten kann und
**R₅** = -Alkyl (substituiert und unsubstituiert), -Alkenyl (substituiert und unsubstituiert), - Alkylaryl (substituiert und unsubstituiert), -Aryl (substituiert und unsubstituiert), substituiertes glycosyl, -NH-Aryl (einfach oder mehrfach substituiert und unsubstituiert), -zyklischer Heteroalkylrest, - zyklischer Rest (einfach oder mehrfach substituiert und unsubstituiert) verbunden mit R₂, - zyklischer Rest (einfach oder mehrfach substituiert und unsubstituiert) verbunden mit R₃ bedeuten kann.

Die genannten Michael-Akzeptoren können sowohl als freie Base wie auch als Salz vorliegen: z.B. als Fluorid, Chlorid, Bromid, lodid, Sulfat, Carbonat, Ascorbat, Acetat oder Phoshat. Im Besonderen als Halogensalze, wie z.B. Chlorid und Bromid.

Erfindungsgemäß ist ferner die Verwendung der vorgenannten Michael-Akzeptoren zur Behandlung und/oder Prophylaxe unerwünschter Hautpigmentierung.

Die Dokumente WO2006078399, JP2010280735, US2008317692, DE102010045890, WO2012098134, WO2004073675, "New assays for the tvrosine hydroxylase and dopa oxidase activities of tyrosinase", "EUROPEAN JOURNAL OF BIOCHEMISTRY, (19910601), vol. 198, no. 2, WO2009099195, M. SEIBERG ET AL., J. CELL. SCI., (2000), vol. 113, Seiten 3093 - 101, A. KLIGMAN, ARCH. DERMATOL., (1975), vol. 111, Seiten 40 - 48, BIOORGANIC & MEDICINAL CHEMISTRY LETTER, (2007), vol. 17, Seiten 6871 - 6875, WINDER, A.J.; HARRIS, H., "New assavs for the tvrosine hydroxylase and dopa oxidase activities of tyrosinase", EUR. J. BIOCHEM., (1991), vol. 198, konnten nicht den Weg zur vorliegenden Erfindung weisen.

### Methodenbeschreibung der Wirksamkeitsuntersuchungen:

Die Wirksamkeit der Michael-Akzeptoren wurde mit einem Enzymtest belegt, in der Umsatz von L-DOPA zu L-Dopachinon durch eine humane Tyrosinase gemessen wurde. Bei dieser literaturbekannten Methode (Winder, A.J. and Harris, H., New assays for the tyrosine hydroxylase and dopa oxidase activities of tyrosinase. Eur. J. Biochem. (1991), 198, 317-26) wird das Reaktionsprodukt L-Dopaquinone mit MBTH (3-methyl-2-benzothiazoline hydrazone) zu einer pinkfarbenen Substanz umgesetzt, deren Zunahme über die Zeit durch Absorption bei 490 nm gemessen wird. In Tabelle ein sind Wirksamkeitsdaten für einige der beanspruchten Substanzen beispielhaft dargestellt. Daraus lässt sich schließen, dass die erfindungsgemä-ßen Substanzen äußerst effektive pigmentierungs-inhibierende Substanzen sind.

| **Tabelle: Inhibition der Tyrosinaseaktivität durch Michael Akzeptoren** | |
|---|---|
| **Substanz** | **EC 50-Wert (µM)** |
| Zinnamaldehyd | 211 |
| Allyl cinnamat | 2340 |
| Vinyl cinnamat | 2610 |
| Ethyl cinnamat | 2590 |
| Benzyl cinnamat | 260 |
| 2-(4-Methyl-1-cyclohexyliden)essigsäure | 6537 |
| Kaliumsorbat | 1526 |
| Isoamyl p-Methoxycinnamat | 358 |
| trans-Ferulasäure | 2215 |
| (E)-3-(3,4-dihydroxyphenyl)acrylsäure | 1366 |
| (Z)-3-(4-(tert-butyl)phenyl)-3-hydroxy-1-(4-methoxyphenyl)prop-2-en-1-on | 1554 |
| (E)-4-(4-hydroxy-3-methoxyphenyl)but-3-en-2-on | 96900 |
| Benzyl 2-hydroxybenzoat | 378 |
| Benzyl benzoat | 998 |
| Natrium Benzoat | 1444 |
| Hexyl 2-hydroxybenzoat | 4230 |
| Methyl 2,5-Dihydroxybenzoat | 2449 |
| Cyclohexyl 2-hydroxybenzoat | 547 |
| 2-Hydroxy-4-methoxy Benzoesäure | 2656 |
| (E)-3-(thiophen-2-yl)acrylsäure | 32 |
| (E)-3-(pyridin-4-yl) acrylsäure | 697 |
| (E)-3-(pyridin-3-yl)acrylsäure | 634 |
| (E)-3-(1H-indol-3-yl)acrylsäure | 833 |
| (E)-3-(furan-2-yl)acrylsäure | 166 |
| 5-Propyl-2-thioxo-2,3-dihydropyrimidin-4(1H)-on | 494 |
| (E)-N-(2-(5-hydroxy-1H-indol-3-yl)ethyl)-3-(4-hydroxyphenyl)acrylamid | 515 |
| 5-(1,2-Dihydroxyethyl)-3,4-dihydroxyfuran-2(5H)-on | 522 |

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-ÖI (W/O) oder vom Typ Öl-in-Wasser (O/W), eine multiple Emulsionen, beispielsweise vom Typ Wasser-in-ÖI-in-Wasser (W/O/W), ein Gel, einen festen Stift, eine Salbe oder auch ein Aerosol darstellen. Es ist auch erfindungsgemäß vorteilhaft, die erfindungsgemäß verwendeten Substanzen und/oder deren Derivate in verkapselter Form darzureichen, z.B. in Kollagenmatrices und anderen üblichen Verkapselungsmaterialien, z.B. als Celluloseverkapselungen, in Gelatine oder liposomal verkapselt.

Es ist auch möglich und vorteilhaft im Sinne der vorliegenden Erfindung, die erfindungsgemäß verwendeten Substanzen und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Feuchthaltemittel wie z.B. Propylenglycol, Panthenol oder Hyaluronsäure sowie insbesondere ein oder mehrere Verdickungsmittel, welches oder welche vorteilhaft gewählt werden können aus der Gruppe Siliciumdioxid, Aluminiumsilikate, Hydroxypropylmethylcellulose, besonders vorteilhaft ein Polyacrylat wie beispielsweise Carbopole Typ 980, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Propyleglycol, und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| O/W-Emulsion | Beispiel 1 | Beispiel 2 | Beispiel 3 |
|---|---|---|---|
| 2-(4-Methyl-1-cyclohexyliden)essigsäure | 0,10 | - | - |
| (E)-N-(2-(5-hydroxy-1H-indol-3-yl)ethyl)-3-(4-hydroxyphenyl)acrylamid | - | 0,10 | - |
| (E)-3-(3,4-dihydroxyphenyl)acrylsäure | - | - | 0,10 |
| Behenylalkohol | 1,20 | 1,20 | 1,20 |
| Cetylalkohol | 2,00 | 2,00 | 2,00 |
| Caprylsäure/Caprinsäuretriglyceride | 2,50 | 2,50 | 2,50 |
| Dicaprylylcarbonat | 2,50 | 2,50 | 2,50 |
| C12-15 Alkylbenzoate | 2,50 | 2,50 | 2,50 |
| Cyclomethicon | 2,10 | 2,15 | 2,15 |
| Dimethicon | 0,35 | 0,35 | 0,35 |
| Glycerylstearatcitrat | 2,00 | 2,00 | 2,00 |
| Glycerin | 8,70 | 8,70 | 8,70 |
| Butyleneglycol | 3,00 | 3,00 | 3,00 |
| Wasser + NaOH | 0,03 | 0,03 | 0,03 |
| Methylparaben | 0,20 | 0,20 | 0,20 |
| Propylparaben | 0,10 | 0,10 | 0,10 |
| Phenoxyethanol | 0,40 | 0,4 | 0,40 |
| Carbomer | 0,10 | 0,10 | 0,10 |
| Natriumpolyacrylate | 0,20 | 0,20 | 0,20 |
| Wasser | ad 100,0000 | | |

## Patentansprüche

1. Nicht-therapeutische Verwendung eines oder mehrerer kosmetisch oder dermatologisch unbedenklicher Michael-Akzeptoren zur Verhinderung, Verminderung oder Prophylaxe der Tyrosinaseaktivität der menschlichen Haut und/oder der Aufhellung der letzteren, wobei der Michael-Akzeptor oder die Michael-Akzeptoren gewählt wird oder werden aus der Gruppe
| |
|---|
| Benzyl cinnamat |
| Isoamyl p-Methoxycinnamat |
| Benzyl 2-hydroxybenzoat |
| Cyclohexyl 2-hydroxybenzoat |
| (E)-3-(thiophen-2-yl)acrylsäure |
| (E)-3-(pyridin-4-yl) acrylsäure |
| (E)-3-(pyridin-3-yl)acrylsäure |
| (E)-3-(1H-indol-3-yl)acrylsäure |
| (E)-3-(furan-2-yl)acrylsäure |
| 5-Propyl-2-thioxo-2,3-dihydropyrimidin-4(1H)-on |

2. Verwendung nach Anspruch 1, **dadurch gekennzeichnet, dass** der Michael-Akzeptor oder die Michael-Akzeptoren in kosmetischen oder dermatologischen Zubereitungen, vorliegen, einer Gesamtmenge an Michael-Akzeptoren von 0,000001 Gew.-% bis 30 Gew.-% entsprechend, vorzugsweise 0,001 bis 15 Gew.-%, insbesondere 0,01 bis 5,0 Gew.-%, bezogen auf das Gesamtgewicht der Zubereitungen.

## Claims

1. Non-therapeutic use of one or more cosmetically or dermatologically safe Michael acceptors for the prevention, reduction or prophylaxis of the tyrosinase activity of human skin and/or the lightening of human skin, wherein the Michael acceptor(s) is/are selected from the group comprising
| |
|---|
| Benzyl cinnamate |
| Isoamyl p-methoxycinnamate |
| Benzyl 2-hydroxybenzoate |
| Cyclohexyl 2-hydroxybenzoate |
| (E)-3-(thiophen-2-yl)acrylic acid |
| (E)-3-(pyridin-4-yl)acrylic acid |
| (E)-3-(pyridin-3-yl)acrylic acid |
| (E)-3-(1H-indol-3-yl)acrylic acid |
| (E)-3-(furan-2-yl)acrylic acid |
| 5-Propyl-2-thioxo-2,3-dihydropyrimidin-4(1H)-one |

2. Use according to Claim 1, **characterized in that** the Michael acceptor(s) is/are present in cosmetic or dermatological preparations corresponding to a total amount of Michael acceptors of 0.000001% by weight to 30% by weight, preferably 0.001 to 15% by weight, especially 0.01 to 5.0% by weight, based on the total weight of the preparations.

## Revendications

1. Utilisation non thérapeutique d'un ou de plusieurs accepteurs de Michael inoffensifs du point de vue cosmétique ou dermatologique pour l'inhibition, la réduction ou la prophylaxie de l'activité tyrosinase de la peau humaine et/ou l'éclaircissement de cette dernière, l'accepteur de Michael ou les accepteurs de Michael étant choisis dans le groupe suivant :
| |
|---|
| cinnamate de benzyle |
| p-méthoxycinnamate d'isoamyle |
| 2-hydroxybenzoate de benzyle |
| 2-hydroxybenzoate de cyclohexyle |
| acide (E)-3-(thiophén-2-yl)acrylique |
| acide (E)-3-(pyridin-4-yl)acrylique |
| acide (E)-3-(pyridin-3-yl)acrylique |
| acide (E)-3-(1H-indol-3-yl)acrylique |
| acide (E)-3-(furan-2-yl)acrylique |
| 5-propyl-2-thioxo-2,3-dihydropyrimidin-4(1H)-one |

2. Utilisation selon la revendication 1, **caractérisée en ce que** l'accepteur de Michel ou les accepteurs de Michael sont présents dans des préparations cosmétiques ou dermatologiques en une quantité totale d'accepteurs de Michael de 0,000001 % en poids à 30 % en poids, de préférence de 0,001 à 15 % en poids, notamment de 0,01 à 5,0 % en poids, par rapport au poids total des préparations.
